# EUROPEAN PATENT SPECIFICATION

(11) **EP 1 435 246 B1**
(45) Date of publication and mention of the grant of the patent: **07.06.2006**
(21) Application number: 03250035.7
(22) Date of filing: 06.01.2003
(51) Int. Cl.: A61L 9/12

(54) **Compact diffuser for volatile material**
Kompakter Diffusor für flüchtige Stoffe
Diffuseur compact pour matière volatile

(43) Date of publication of application: 07.07.2004
(73) Proprietor: Lumica Corporation, Koga City, Fukuoka 811-3136 (JP)
(72) Inventor: Harada, Shiro, Koga City, Fukuoka 811-3136 (JP); Fujita, Masahiko, Koga City, Fukuoka 811-3136 (JP)
(74) Representative: Gallafent, Richard John

(56) References cited:
- US-A- 2 395 109
- US-A- 3 856 142
- US-A1- 2002 109 013

## Description

This present invention relates to a compact diffuser for volatile material, such as a volatile aromatic or chemical liquid, capable of diffusing aroma or chemicals, for example an insecticide, mothproofing agent or insect repellent, into the surrounding air.

Such volatile materials are conventionally prepared by dissolving a blended aromatic substance of a natural animal- or vegetable-based aromatic substance and a synthetic aromatic substance in a solvent, commonly an alcohol solvent. Preferably polyhydric alcohols are used to control the volatilization of the aromatic substance, for example, dipropylene glycol, hexylene glycol, propylene glycol and 1,3-butane diol, used singly or in admixture.

Diffusers for such volatile materials are known. Among these known diffusers are disposable diffusers having a vapour-permeable hollow casing made of ethylene-vinyl acetate (EVA) resin, the volatile material being enclosed in the interior of the casing. In this type of diffuser, the volatile constituent of the liquid tends to diffuse into the surrounding air through the casing by taking advantage of vapor permeability derived from the molecular structure of EVA. Such disposable diffusers can have a compact and simplified structure, but it can take several hours to allow the volatile constituent to diffuse into the surrounding air through the EVA resin casing. This slow response is defective from the viewpoint of practicability. In addition, the casing tends to become undesirably deformed due to the reduced volume of the liquid caused by the volatilization of the volatile constituent, resulting in a deterioration in its appearance.

US-A-2395109 discloses an inhaler unit having a flexible casing surrounding an elongate breakable capsule with an absorbent member between the capsule and the casing.

The present invention seeks to provide a compact diffuser for volatile material such as a volatile aromatic or chemical liquid which overcomes the above-noted disadvantages and is capable of promptly diffusing the volatile constituent of the liquid into the surrounding air in a simple operation as the need arises, while preferably preventing any needless volatilisation of the liquid even during a long-term storage, and being of simple construction.

According to the present invention, these problems are solved by the characterising features of the main claim.

Generally, the present invention provides a diffuser for volatile material, such as a volatile aromatic or chemical liquid, comprising a flexible casing defining an inner space and having a vent hole formed therein to provide fluid communication between the inner space and the surrounding air. The flexible casing contains a vapor-impermeabie and breakable capsule hermetically enclosing a volatile liquid, and a liquid-absorbent member adapted temporarily to restrain the outflow of the volatile liquid caused by breakage of the capsule. The liquid absorbent member is located in the interior space between the capsule and the vent hole so as to cover the vent hole, and a cover member is provided which includes a receiving space for detachably receiving the flexible casing, and is formed with a second vent hole for providing fluid communication between the space inside the cover member and the surrounding air.

The cover member may be provided with an engagement device, such as a hook, pin, clip or suction device, for detachably attaching the cover member to an ornamental article such as an earring, finger ring, wristband, necklace or pendant, or to a structural member or to a useful article, for example a writing instrument, mobile telephone or mobile telephone case, e.g. to the conventional flexible handle.

The flexible casing is preferably generally cylindrical and may be made of flexible material such as polypropylene, polyethylene, nylon, or polyvinyl chloride. It may transparent, opaque or coloured.

While the flexible casing is conveniently formed in a cylindrical shape, any suitable shape may be used according to the intended purpose. The flexible casing may have one vent hole or multiple vent holes.

The number and/or dimension of the vent holes may be selectively determined in consideration of the volatilisation speed of the volatile constituent of the aromatic or chemical liquid, and will vary according to the intended purpose. The volatilisation speed may also be varied depending on the properties of the volatile aromatic or chemical liquid in question, by adding an appropriate retarding agent to the volatile aromatic or chemical liquid e.g. to provide a constant volatilisation speed and to maintain the diffusion effect for an intended time period.

The volatile chemical is typically an insecticide, mothproofing agent or insect repellent. Suitable insecticides include, but not limited to, pyrethroids e.g. synthetic pyrethrin and pyrethrum. The pyrethroid may also be an allethrin, phthalthrin or resmethrin. Suitable mothproofing agents or insect repellents include, but are not limited to, dimethyl phthalate ester, 2-ethyl-1,3-hexanediol, dimethyl cis-bicyclo(2,2,1)-5-heptene-2,3-dicarboxylate, and N,N-diethyl succinamic acid N-propyl ester. Other mothproofing agents which may be included are known mothproofing agents for fabrics. The volatile aromatic liquid is used as a volatile substance such as perfume or cologne capable of emitting a pleasant or comfortable odour.

The capsule is made of vapour-impermeable and breakable material, preferably glass. The capsule wall should be designed to have a thickness enabling a user to break it with his/her hand. For example, the wall thickness of a glass capsule may be set in the range of 0.1 to 0.4 mm.

Using a capsule made of vapour-impermeable material such as glass makes it possible to ensure stability of the volatile material even during a long-term storage under high temperature and/or humid conditions.

The liquid-absorbent member is made of vapour-permeable/liquid-absorbent material such as a natural or synthetic fibrous material, or a natural or synthetic porous material and temporarily hoids the outflow of the volatile material once the capsule is broken.

The liquid-absorbent member may be disposed adjacent to the vent hole or wound around the capsule. When the capsule is broken, the liquid-absorbent member can prevent the resulting outflow of volatile material from draining directly from the inner space to the outside through the vent hole. A further advantage is that, even if the capsule is broken into very small pieces, the liquid-absorbent member can prevent such fragments from escaping through the vent hole.

If the flexible casing is cylindrical, it preferably has a length of 20 to 200 mm, an outer diameter of 3 to 12 mm, and a thickness of 0.2 to 3 mm. The vent hole may be formed on the or both ends of such a cylindrical flexible casing. The flexible casing contains a liquid-absorbent member in its inner space.

The volatile material may be hermetically enclosed in the capsule, for example, by preparing a cylindrical elongate ampoule having a planar opening, introducing the volatile material into the ampoule through the planar opening, and immediately thermally melting and closing the opening. In this case, the fused closed end of the capsule has a cuspidate shape.

To use the diffuser according to the present invention, the user bends the flexible casing by his/her hand to break the capsule. This breaking operation may be facilitated if the flexible casing is of cylindrical elongated shape. Upon the breakage of the capsule, the volatile liquid runs out of the capsule into the inner space. The outflow of the volatile liquid is temporarily restrained by the liquid-absorbent member, and it then gradually volatilised and diffuses from the liquid-absorbent member to the surrounding air through the vent hole.

To further illustrate and explain the invention, reference is made to the accompanying drawings, which show, by way of example, various embodiments of the present invention. in the drawings:
Figure 1 is an explanatory sectional view of a diffuser for volatile material for use in various embodiments of the present invention;
Figure 2 is an explanatory view of a pendant type diffuser according to a first embodiment of the present invention;
Figure 3 is an explanatory view of a pencil type diffuser according to a second embodiment of the present invention;
Figure 4 is an explanatory view of a wristband type diffuser not according to the present invention;
Figure 5 is an explanatory view of a mothproofing diffuser according to a fourth embodiment of the present invention.;
Figure 6 is an explanatory view of an aroma diffuser for use in a bathroom according to a fifth embodiment of the present invention; and
Figure 7 is an explanatory view of an ornamental diffuser used with a portable phone according to a sixth embodiment of the present invention.

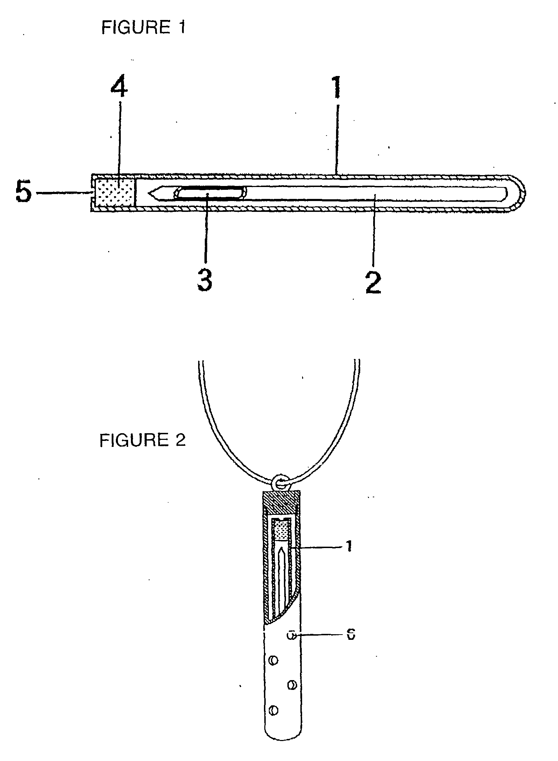

Figure 1 shows a diffuser for volatile material embodiments of the present invention. It consists of a cylindrical flexible casing 1 in which is located a glass capsule 2 containing volatile material 3. A liquid-absorbent member 4 lies adjacent a vent hole 5 in the end of casing 1.

Figure 2 shows a pendant type diffuser according to a first embodiment of the present invention. The flexible casing is surrounded by a cylindrical cover member made of metal or plastic material and formed with a plurality of vent holes 6.

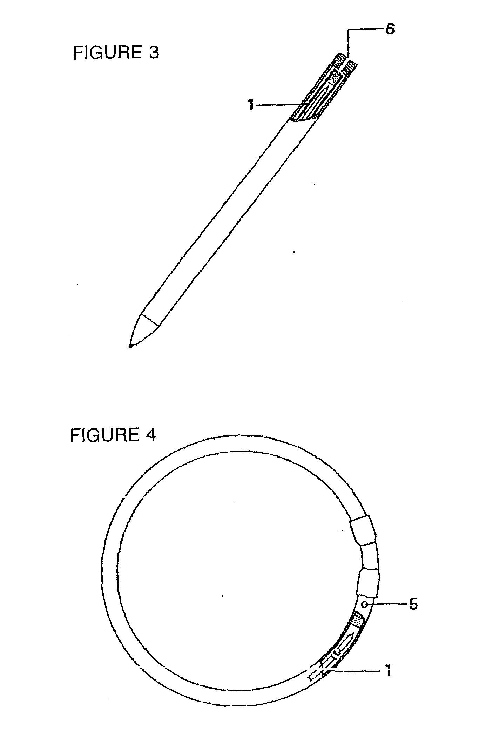

Figure 3 is an explanatory view of a diffuser according to a second embodiment of the present invention in which the diffuser includes a cover member formed as a pencil. The cover member has a receiving space for receiving the flexible casing 1 and a cap formed with a vent hole 6.

Figure 4 shows a wristband type diffuser not according to the present invention. In this embodiment, the diffuser includes a pipe-shaped flexible polyethylene casing having an outer diameter of 5 mm and a length of 180 mm, and a joint for coupling both ends of the flexible casing. The flexible casing 1 contains the liquid-impermeable and breakable capsule enclosing a volatile aromatic liquid, and the liquid-absorbent member as in the embodiment shown in Figure 1. A vent hole 5 is formed at one end of the flexible casing 1 and covered by the liquid-absorbent member. When diffuser is used, the flexible casing is bent to break the capsule, and both ends of the flexible casing are coupled by the joint to use as a wristband. This diffuser may be sold as a kit of parts which are to be combined to form an ornament, the kit consisting of a joint piece and the flexible casing 1 with the capsule and the liquid-absorbent member.

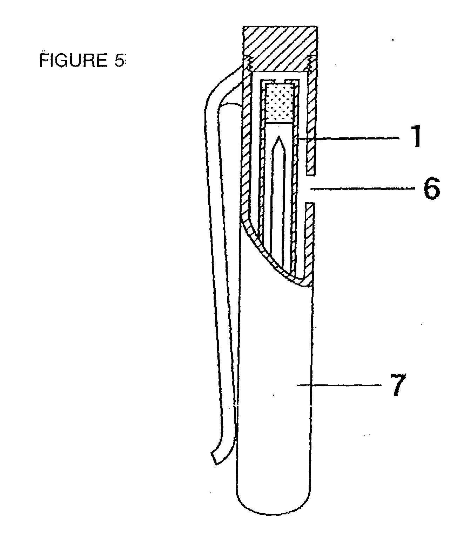

Figure 5 is a part sectional view of a mothproofing diffuser according to a fourth embodiment of the present invention. In this embodiment, the diffuser includes a cover member 7 having a receiving space, a vent hole 6 and a detachable cap, and a clip provided on the side opposite the vent hole 6. The flexible casing 1 in the space in cover member 7 contains the liquid-impermeable and breakable capsule enclosing a volatile mothproofing agent, and the liquid-absorbent member.

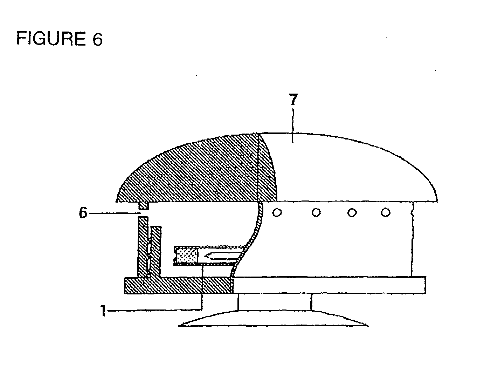

Figure 6 is an explanatory view of an aroma diffuser for use in a bathroom or toilet according to a fifth embodiment of the present invention. In this embodiment, the diffuser includes a cover member 7 having an interior space, a plurality of vent holes 6, and a detachable cap, and a suction cup provided on the opposite side of the cap, which cup acts as a foot and enables the diffuser to be attached to a tiled wall if desired.

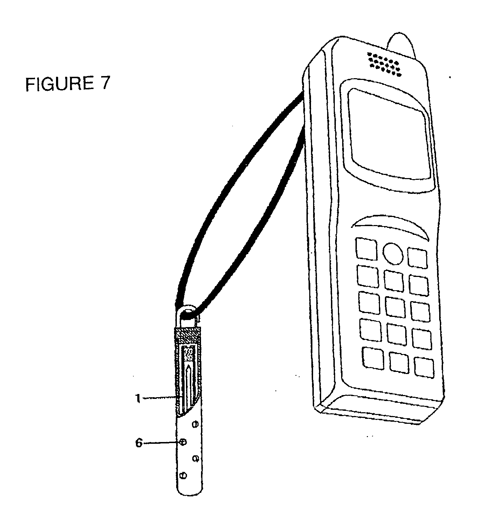

Figure 7 shows an ornamental diffuser according to a sixth embodiment of the present invention used with a portable phone. The diffuser which contains a flexible casing 1 inside a case with vent holes 6 can be detachably attached to a cord which is attached to a portable phone by means of a hook on one end of the cover member.

These and other embodiments of the present invention provide compact diffusers capable of promptly diffusing the volatile constituent of the liquid into the surrounding air in a simple operation as the need arises while maintaining a stable volatilization of the liquid with extended effective life. The diffusers may be stored for a long term without deterioration in quality, for ornamental or practical applications. Further, the diffuser can be used flexibly according to the various intended purposes by preparing a plurality of capsules enclosing different kinds of volatile aromatic or chemical liquids.

## Claims

1. A diffuser for volatile material, comprising a flexible casing (1) defining an interior space and having a vent hole (5) formed therein to provide fluid communication between the interior space and the surrounding air, a vapour-impermeable and breakable capsule (2) hermetically enclosing a volatile material (3) located in the interior space and a liquid-absorbent member (4) adapted to temporarily hold the outflow of the volatile material caused by breakage of the capsule (2), the liquid absorbent member (4) being located in the interior space between the capsule (2) and the vent hole (5) so as to cover the vent hole (5) and **characterised by** a cover member including a receiving space for detachably receiving the flexible casing, the cover member being formed with a second vent hole (6) for providing fluid communication between the space inside the cover member and the surrounding air.

2. A diffuser according to Claim 1, wherein the volatile material is a volatile aromatic liquid including perfume aroma, fragrance or incense, or a volatile chemical liquid insecticide, mothproofing agent or insect repellent.

3. A diffuser according to Claim 1 or 2, wherein the capsule (2) is breakable by applying a bending force to the flexible outer wall of the casing (1).

4. A diffuser according to any one of Claims 1 to 3 and configured as an earring, finger ring, wristband (Figure 4), necklace, pendant (Figure 2) or portable phone leash (Figure 7).

5. A diffuser according to any one of Claims 1 to 4 wherein the cover member includes an engagement device for detachably attaching the cover member to an ornamental article or a structural member.

6. A diffuser according to Claim 5 wherein the engagement device is a hook, pin, clip, suction cup or non-slip device.

## Patentansprüche

1. Zerstäuber für einen flüchtigen Stoff, mit einem flexiblen Gehäuse (1), das einen Innenraum aufweist und in dem ein Lüftungsloch (5) ausgebildet ist, um eine Fluidverbindung zwischen dem Innenraum und der umgebenden Luft bereitzustellen, einer dampfundurchlässigen und aufbrechbaren Kapsel (2), die einen in dem Innenraum angeordneten flüchtigen Stoff (3) hermetisch einschließt, und einem flüssigkeitsabsorbierenden Element (4), das zum vorübergehenden Halten des Ausströmens des flüchtigen Stoffes, verursacht durch das Aufbrechen der Kapsel (2), ausgebildet ist, wobei das flüssigkeitsabsorbierende Element (4) in dem Innenraum zwischen der Kapsel (2) und dem Lüftungsloch (5) derart angeordnet ist, daß das Lüftungsloch (5) abgedeckt ist, **gekennzeichnet durch** ein Abdeckelement mit einem Aufnahmeraum zum Aufnehmen und wieder Entfernen des flexiblen Gehäuses, wobei das Abdeckelement mit einem zweiten Lüftungsloch (6) versehen ist, um eine Fluidverbindung zwischen dem Raum innerhalb des Abdeckelements und der umgebenen Luft bereitzustellen.

2. Zerstäuber nach Anspruch 1, worin der flüchtige Stoff eine flüchtige aromatische Flüssigkeit mit einem Parfumaroma, einem Riechstoff oder einem Räuchermittel, oder ein flüchtiges chemisches flüssiges Insektizid, ein Mottenschutzmittel oder ein Insektenabwehrmittel ist.

3. Zerstäuber nach Anspruch 1 oder 2, worin die Kapsel (2) durch Anwenden einer Biegekraft auf die flexible Außenwand des Gehäuses (1) zerbrechbar ist.

4. Zerstäuber nach einem der Ansprüche 1 bis 3, ausgebildet als Ohrring, Fingerring, Armband (Figur 4), Halsband, Anhänger (Figur 2) oder Band für ein Mobiltelefon (Figur 7) .

5. Zerstäuber nach einem der Ansprüche 1 bis 4, worin das Abdeckelement eine Eingriffsvorrichtung zum abnehmbaren Befestigen des Abdeckelements an einem Ziergegenstand oder einem Konstruktionselement aufweist.

6. Zerstäuber nach Anspruch 5, worin die Eingriffsvorrichtung ein Haken, ein Stift, eine Klammer, ein Saufnapf oder eine gleitfeste Vorrichtung ist.

## Revendications

1. Diffuseur pour matériau volatil, comprenant un boîtier souple (1) définissant un espace intérieur et comportant un trou de mise à l'air libre (5) formé dans celui-ci pour permettre une communication de fluide entre l'espace intérieur et l'air environnant, une capsule imperméable à la vapeur et cassable (2) enfermant de façon hermétique un matériau volatil (3), située dans l'espace intérieur et un élément absorbant les liquides (4) conçu pour retenir temporairement l'écoulement du matériau volatil provoqué par la rupture de la capsule (2), l'élément absorbant les liquides (4) étant positionné dans l'espace intérieur entre la capsule (2) et le trou de mise à l'air libre (5) de façon à couvrir le trou de mise à l'air libre (5) et **caractérisé par** un étui comprenant un espace de réception destiné à recevoir de façon amovible le boîtier souple, l'étui étant formé d'un second trou de mise à l'air libre (6) destiné à permettre une communication de fluide entre l'espace à l'intérieur de l'étui et l'air environnant.

2. Diffuseur selon la revendication 1, dans lequel le matériau volatil est un liquide aromatique volatil comprenant un arôme agréable, du parfum ou de l'encens, ou un insecticide, agent anti-mites ou insectifuge liquide chimique volatil.

3. Diffuseur selon la revendication 1 ou 2, dans lequel la capsule (2) peut être cassée en appliquant une force de flexion à la paroi extérieure souple du boîtier (1).

4. Diffuseur selon l'une quelconque des revendications 1 à 3 et configuré sous forme d'une boucle d'oreille, d'une bague, d'un bracelet (figure 4), d'un collier, d'un pendentif (figure 2) ou pour une sangle de téléphone portable (figure 7).

5. Diffuseur selon l'une quelconque des revendications 1 à 4, dans lequel l'étui comprend un dispositif de couplage destiné à fixer de façon amovible l'élément de protection à un article de décoration ou un élément structurel.

6. Diffuseur selon la revendication 5, dans lequel le dispositif de couplage est un crochet, une broche, une attache, une ventouse ou un dispositif antidérapant.
